(19) **Europäisches Patentamt / European Patent Office / Office européen des brevets**

(11) **EP 3 207 377 B1**

(12) ## EUROPEAN PATENT SPECIFICATION

(45) Date of publication and mention
of the grant of the patent:
**06.11.2024  Bulletin 2024/45**

(51) International Patent Classification (IPC):
**G01N 33/574** *(2006.01)*

(21) Application number: **15850076.9**

(22) Date of filing: **13.10.2015**

(52) Cooperative Patent Classification (CPC):
**G01N 33/57488; G01N 33/57423;** G01N 2800/52;
G01N 2800/56

(86) International application number:
**PCT/US2015/055275**

(87) International publication number:
**WO 2016/061064 (21.04.2016 Gazette 2016/16)**

(54) **CIRCULATING TUMOR CELL DIAGNOSTICS FOR THERAPY TARGETING PD-L1**

DIAGNOSE ZIRKULIERENDER TUMORZELLEN FÜR AUF PD-L1-ABZIELENDE THERAPIE

DIAGNOSTIC DE CELLULES TUMORALES CIRCULANTES POUR UNE THÉRAPIE CIBLÉE À PD-L1

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.10.2014  US 201462064320 P**

(43) Date of publication of application:
**23.08.2017  Bulletin 2017/34**

(73) Proprietor: **Epic Sciences, Inc.**
**San Diego, CA 92121 (US)**

(72) Inventor: **DITTAMORE, Ryan**
**San Diego, CA 92121 (US)**

(74) Representative: **Jones Day**
**Rechtsanwälte, Attorneys-at-Law, Patentanwälte**
**Prinzregentenstrasse 11**
**80538 München (DE)**

(56) References cited:
**WO-A2-2014/151006     WO-A2-2014/151006**

- **BENJAMIN K GIBBS ET AL: "Abstract 4816: Development of an integrated analysis platform of circulating melanoma cells for PD-L1 expression as a predictive biomarker", CANCER RESEARCH, 5 April 2014 (2014-04-05), XP055460476, Retrieved from the Internet <URL:http://cancerres.aacrjournals.org/content/74/19_Supplement/4816> [retrieved on 20180319]**

- **MARIO GIULIANO ET AL: "Circulating tumor cells as early predictors of metastatic spread in breast cancer patients with limited metastatic dissemination", BREAST CANCER RESEARCH, CURRENT MEDICINE GROUP LTD, GB, vol. 16, no. 5, 16 September 2014 (2014-09-16), pages 440, XP021200517, ISSN: 1465-5411, DOI: 10.1186/S13058-014-0440-8**

- **H. BELTRAN ET AL: "The Initial Detection and Partial Characterization of Circulating Tumor Cells in Neuroendocrine Prostate Cancer", CLINICAL CANCER RESEARCH, vol. 22, no. 6, 15 March 2016 (2016-03-15), US, pages 1510 - 1519, XP055382275, ISSN: 1078-0432, DOI: 10.1158/1078-0432.CCR-15-0137**

- **PAVLOS MSAOUEL ET AL: "Diagnostic value of circulating tumor cell detection in bladder and urothelial cancer: systematic review and meta-analysis", BMC CANCER, BIOMED CENTRAL, LONDON, GB, vol. 11, no. 1, 4 August 2011 (2011-08-04), pages 336, XP021105122, ISSN: 1471-2407, DOI: 10.1186/1471-2407-11-336**

- **ARCHANA ANANTHARAMAN ET AL: "Programmed death-ligand 1 (PD-L1) characterization of circulating tumor cells (CTCs) in muscle invasive and metastatic bladder cancer patients", BMC CANCER, vol. 16, no. 1, 22 September 2016 (2016-09-22), XP055712253, DOI: 10.1186/s12885-016-2758-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- WENDEL, M ET AL.: "Fluid biopsy for Circulating Tumor Cell identification In Patients With Early And Late Stage Non-Small Cell Lung Cancer;", A GLIMPSE INTO LUNG CANCER BIOLOGY. PHYS. BIOL.., vol. 9, no. 1, February 2012 (2012-02-01), pages 016005, XP020218198
- JILAVEANU, LB ET AL.: "PD-L1 Expression in Clear Cell Renal Cell Carcinoma: An Analysis of Nephrectomy and Sites of Metastases.", J. CANCER., vol. 5, no. 3, 24 January 2014 (2014-01-24), pages 166 - 172, XP055182028
- ZHENG, Z ET AL.: "Level of circulating PD-L1 expression in patients with advanced gastric cancer and its clinical implications.", CHINESE J. CANCER RES., vol. 26, no. 1, 1 February 2014 (2014-02-01), pages 104 - 111, XP055429369

**Description**

The invention relates generally to the field of cancer diagnostics and, more specifically to methods for determining if a subject afflicted with bladder cancer is at risk for metastasis.

**BACKGROUND**

Cancer is traditionally treated with either conventional therapy, including chemotherapy and radiation therapy, or targeted drugs that directly kill tumor cells. Immunotherapy has become an increasingly appealing therapeutic strategy for patients with cancer, with many late-stage clinical trials demonstrating overall survival (OS) advantages in melanoma and castrationresistant prostate cancer. More recently, non-small cell lung cancer (NSCLC) has become a focus for the next generation of immune-based therapeutic strategies. Immunotherapy, in particular the use of monoclonal antibodies that block inhibitory immune checkpoint molecules and therefore enhance the immune response to tumors, has shown clinical promise in advanced solid tumors.

Immunotherapy targeting the Programmed death-1 (PD-1) / Programmed death-ligand 1 (PD-L1) pathway will become an integral part of the clinical management strategy for solid tumors. PD-1 is a co-receptor that is expressed predominantly by T cells. The binding of PD-1 to its ligands, PD-L1 or PD-L2, is vital for the physiologic regulation of the immune system. A major functional role of the PD-1 signaling pathway is the inhibition of self-reactive T cells, which serve to protect against autoimmune diseases. Elimination of the PD-1 pathway can therefore result in the breakdown of immune tolerance that can ultimately lead to the development of pathogenic autoimmunity. Conversely, tumor cells can at times co-opt the PD-1 pathway to escape from immuno surveillance mechanisms. Therefore, blockade of the PD-1 pathway has become an attractive target in cancer therapy. Recent clinical trials have shown that anti-PD-1 agents have profound effects on solid tumor regression. Upregulation of PD-L1 expression levels has been demonstrated in many different cancer types (e.g., melanoma $[40\%-100\%]$, NSCLC $[35\%-95\%]$, and multiple myeloma [93%]), and high levels of PD-L1 expression have been linked to poor clinical outcomes. Current approaches include six agents that are either PD-1 and PD-L1 targeted neutralizing antibodies or fusion proteins. More than forty clinical trials are underway to better define the role of PD-1 blockade in variety of tumor types.

Circulating tumor cells (CTCs) represent a significant advance in cancer diagnosis made even more attractive by their non-invasive measurement. Cristofanilli et al., N Engl J Med 351:781-91, (2004) showed that CTCs released from either a primary tumor or its metastatic sites hold important information about the biology of the tumor. Quantifying and characterizing CTCs, as a liquid biopsy, assists clinicians in selecting the course of therapy and monitoring how a patient's cancer evolves. CTCs can therefore be considered not only as surrogate biomarkers for metastatic disease but also as a promising key tool to track tumor changes, treatment response, cancer recurrence or patient outcome non-invasively. Historically, the extremely low levels of CTCs in the bloodstream combined with their unknown phenotype have significantly impeded their detection and limited their clinical utility. A variety of technologies are presently being developed for detection, isolation and characterization of CTCs in order to utilize their information. Msaouel and Koutsilieris, BMC Cancer (11): 336 (2011) relates to a systemic review and meta-analysis of the diagnostic value and prognostic significance of circulating tumor cell (CTC) detection in patients with bladder cancer and other urothelial cancers.

A need exists to develop accurate and non-invasive methods for prospective identification of patients that are candidates for PD-1/PD-L1 immunotherapy. The present invention addresses this need by providing a method for determining if a subject afflicted with cancer is a candidate for PD-L1 targeted immunotherapy based on a robust CTC detection and characterization platform that enables phenotypic and genotypic characterization of CTCs. Related advantages are provided as well.

**SUMMARY OF THE INVENTION**

The invention is set out in the appended claim. Generally, the present disclosure describes methods for determining if a subject afflicted with cancer is a candidate for Programmed Death Ligand-1 (PD-L1) targeted immunotherapy, which method comprises (a) providing a liquid biopsy sample obtained from the subject afflicted with cancer; (b) detecting CTCs in the liquid biopsy sample; (c) calculating what proportion of CTCs in the liquid biopsy express PD-L1; and (c) identifying the subject as a candidate for PD-L1 targeted immunotherapy based on an assessment that the proportion of the CTCs in the liquid biopsy that express PD-L1 exceeds a pre-determined threshold level.

The invention provides a method for determining if a subject afflicted with bladder cancer is at risk for metastasis, wherein the method comprises (a) detecting circulating tumor cells (CTCs) that express PD-L1 in a liquid biopsy sample from the subject afflicted with bladder cancer; (b) calculating what proportion of CTCs in the liquid biopsy that express PD-L1 is cytokeratin (CK)-negative; and (c) identifying the subject as a candidate at risk for metastasis based on an assessment that the proportion of CK-negative CTCs in the liquid biopsy that express PD-LI exceeds a predetermined threshold level. Other features and advantages of the invention will be apparent from the detailed description, and from the claim.

## BRIEF DESCRIPTION OF THE DRAWINGS

Figure 1 shows feasibility of PD-L1 measurement on tumor cells detected in whole blood. PD-L1 expression of cell lines spiked into donor blood and detected utilizing the Epic process described herein. Each dot represents a unique cell identified. PD-L1 IF assay: control cell lines - Colo205 (negative), A549 (heterogeneous expression), SU-DHL-1 (positive - medium expression, CK-). Higher expression of PD-L1 is observed in cell lines known to overexpress PD-L1.

Figure 2 shows imagery of PD-L1 expression on tumor cell lines. PD-L1 protein is observed on the cell membrane of tumor cells. The Epic assay described herein demonstrates protein expression patterns associated with cell membrane expression of PD-L1 and confirm assay specificity. Figure 2 shows representative images of cell line cells determined using the PD-L1 IF assay.

Figures 3A and 3B show PD-L1 expression induced by interferon gamma (IFNg). IFNg is known to increase PD-L1 expression on tumor cells. Cell lines treated with and without IFNg were spiked into whole blood and analyzed on the Epic platform described herein. Increased PD-L1 expression is confirmed with IFNg treated samples. Figure 3A shows PD-L1 on IFNg treated cells. Figure 3B shows un-treated and IFNg treated-combined data (1/2000 dilution of anti-PD-L1) (groups of un-treated and treated, from left to right, Colo205, A549, SU-DHL-1).

Figure 4 and Figure 5 show that lung cancer CTCs can express PD-L1. Figure 4 (PD-L1 translated into NSCLC patient with ADC stage 1A) and Figure 5 (PD-L1 translated into NSCLC ADC stage 3A) show immunofluorescence images of traditional CTCs detected in non-enriched blood samples of lung cancer patients, and PD-L1 protein expression is seen in both early and late stage NSCLC. The columns show composite images (left column); DAPI staining (second column); CK-staining (third column); CD45-staining (fourth column) and PD-L1 staining (right column).

Figure 6 shows that bladder cancer CTCs can express PD-L1. Figure 6 shows PD-L1 protein expression is seen in traditional CTCs (CK+, CD45-), CTC Clusters, and CK-CTCs. Each dot represents a unique cell identified.

Figure 7 shows that bladder cancer CTCs can express PD-L1 in low CK expressing CTCs. The CK-CTCs (seen in purple, below the dotted line) demonstrate cells with epithelial plasticity and PD-L1 positivity. Each dot represents a unique cell identified.

Figure 8 shows a bladder cancer patient case study (fully classified (1st & 2nd pass complete), Patient UCO03-B011). Many CTCs expressing PD-L1 express low levels of CK. Each dot represents a unique cell identified.

Figure 9 shows that PD-L1 expression is seen in WBCs at varying levels. PD-L1 expression is observed in both patient samples and cell-line spiked heatlhy donor blood (ninth and tenth columns).

Figure 10 and Figure 11 show imagery of WBC PD-L1 expression in non-enriched blood samples from bladder cancer patients. Cell memberane PD-L1 expression is consistently observed in patient WBCs (fifth column). Figure 10, PD-L1+ WBCs, patient UCO03-B011. Figure 11, PD-L1+ WBCs, patient UCO03-B007.

Figure 12, Figure 13, and Figure 14 show imagery of CTC PD-L1 expression in bladder cancer patient samples. Cell memberane PD-L1 expression is consistently observed in patient CTCs (fifth column). Figure 12, PD-L1+ CTCs, patient UCO03-B011. Figure 13, PD-L1+ CTCs, patient UCO03-B011. Figure 14, PD-L1+ candidate CTCs, patient UCO03-B011.

Figure 15 shows ROC curve analysis. Figure 15 shows PD-L1 expression observed in known PD-L1 positive and PD-L1 negative cell lines spiked in healthy donor blood, demonstrating the sensitivity and specificity of the assay.

Figure 16 shows optimum PD-L1 cutoff. In Figure 16, utilizing sensitivity and specificity, PD-L1 positivity cutoffs were generated and chosen for optimal separation.

## DETAILED DESCRIPTION

*[insert here from new page 5a]

The present disclosure is based, in part, on the identification of PD-L1, a biomarker for cancer immunotherapy, on CTCs. The present disclosure is more specficially based on the identification of PD-L1 expression on CTCs, including cytokeratin-positive (CK+) and cytokeratin-negative (CK-) CTCs, and white blood cells (WBCs). The identification of PD-L1 on CTCs, including cytokeratin-positive (CK+) and cytokeratin-negative (CK-) CTCs, enables a novel method for identifying patients that are candidates for PD-L1 targeted immunotherapies. In addition, the ability to identify CTCs that are CK- by detecting PD-L1 expression provides a novel mechanism to identify non-traditional CTCs in a patient. Furthermore, the present methods enable identification of patients likely to benefit or show resistance to PD-1/PD-L1 targeted immunotherapies across multiple cancer types based on the enumeration and characterization of one or more of the following parameters: CK+ CTCs, CK- CTC , CK+/CK- CTC ratio, WBC identification based on CD45 and PD-L1 protein expression within CTC, CTC subpopulations and WBCs. The algorithm provides an output that can be utilized to identify patients who are likely to benefit from or have resistance to PD-1 and PD-L1 targeted immunotherapies across multiple cancer types. Additionally, the identification of novel CK-/PD-L1+ CTCs in patient samples may be utilized for the predictive, prognostic, or therapy monitoring strategies.

The clinical rationale for targeting the PD-1/PD-L1 pathway is sound. PD-1 is a T-cell molecule that binds to the ligands PD-L1 or PD-L2. PD-L1 is typically expressed on tumor cells and is induced by gamma interferon secreted by activated T cells. Briefly, the activated T cells that could kill tumors are specifically disabled by those tumors that express PD-L1, which binds to PD-1, and creates a phenotype known as T-cell exhaustion. Clinical data from studies of antibodies directed against PD-1 and PD-L1 have shown encouraging safety profiles and remarkable antitumor activity in subsets of patients with metastatic disease, including malignancies (such as lung cancer) that were previously thought to be unresponsive to immunotherapy.

The methods disclosed herein enable patient selection for PD-1 and PD-L1 therapies, as well as pharmacodynamic measurements of efficacy of PD-1 and PD-L1 therapies and identification of mechanisms of resistance to non- PD-1/PD-L1 targeted therapies. The methods disclosed herein further disclose performance of a liquid biopsy to Provided herein is a method for determining if a subject afflicted with bladder cancer is at risk for metastasis, which method comprises: (a) detecting circulating tumor cells (CTCs) that express PD-L1 in a liquid biopsy sample from the subject afflicted with bladder cancer; (b) calculating what proportion of CTCs in the liquid biopsy that express PD-L1 are cytokeratin (CK)-negative; and (c) identifying the subject as a candidate at risk for metastasis based on an assessment that the proportion of CK-negative CTCs in the liquid biopsy that express PD-L1 exceeds a predetermined threshold level. identify clones missampled by a prior tissue biopsy, to monitor disease status and/or gain diagnostic information on patients that are not candidates for an invasive biopsy.

A high incidence of CK- CTCs is associated with increased risk for development of new patient metastasis. The methods disclosed herein enable detection of CK- CTC subpopulations and consequently the identification of patients with high CK- CTC counts that are not likely to respond to targeted treatment. The methods disclosed herein to identify CK- CTCs and to characterize morphometric and genotypic parameters in this important subpopulation can inform diagnostics strategies for patient selection, pharmacodynamics monitoring and mechanisms of disease resistance.

The present disclosure provides a method for determining if a subject afflicted with bladder cancer is a candidate for PD-L1 targeted immunotherapy, which method comprises (a) providing a liquid biopsy sample obtained from the subject afflicted with cancer; (b) detecting CTCs in the liquid biopsy sample; (c) calculating what proportion of CTCs in the liquid biopsy express PD-L1; and (d) identifying the subject as a candidate for PD-L1 targeted immunotherapy based on an assessment that the proportion of the CTCs in the liquid biopsy that express PD-L1 exceeds a pre-determined threshold level. In some embodiments, the method for determining if a subject afflicted with bladder cancer is a candidate for PD-L1 targeted immunotherapy further comprises assessing morphological characteristics of the CTCs. In certain embodiments, the method for determining if a subject afflicted with bladder cancer is a candidate for PD-L1 targeted immunotherapy further comprises determining the genotype of the CTCs.

The method for determining if a subject afflicted with cancer is a candidate for PD-L1 targeted immunotherapy may comprise identification of white blood cells (WBCs) in the liquid biopsy sample, for example, by utilizing CD45. The method for determining if a subject afflicted with cancer is a candidate for PD-L1 targeted immunotherapy comprises enumeration of WBCs in the liquid biopsy sample and determination of what proportion of WBCs that express PD-L1. The method for determining if a subject afflicted with cancer is a candidate for PD-L1 targeted immunotherapy may comprise determining proportion of CTCs that express PD-L1 is cytokeratin-negative (CK-negative).

Also, the method for determining if a subject afflicted with cancer is a candidate for PD-L1 targeted immunotherapy may employ an algorithm for identification of the subject as a candidate for PD-L1 targeted immunotherapy. The method may identify a candidate for Programmed Death-1 (PD-1) targeted immunotherapy.

The method for determining if a subject afflicted with cancer is a candidate for PD-L1 targeted immunotherapy may comprise a step of communicating the determination to a health care provider, for example, that the subject was not identified as a candidate based on the assessment that the proportion of CTCs in the liquid biopsy that express PD-L1 is below a predetermined threshold level. The method for determining if a subject afflicted with cancer is a candidate for PD-L1 targeted immunotherapy comprises a step of communicating the determination to a health care provider, for example, that the subject was identified as a candidate based on the assessment that the proportion of CTCs in the liquid biopsy that express PD-L1 is above a predetermined threshold level. Also, the method for determining if a subject afflicted with cancer may be a candidate for PD-L1 targeted immunotherapy comprises a final step administering a composition of a therapeutically effective amount of an agent that potentiates an endogenous immune response in the candidate. The agent may inhibit signaling from an inhibitory immunoregulator such as, for example, a component of a PD-1/PD-L1 signaling pathway.

Also described is a method of monitoring expression of PD-L1 on circulating tumor cells (CTCs) of a subject undergoing PD-L1 targeted immunotherapy, which method comprises (a) providing at least two liquid biopsy samples from the subject undergoing PD-L1 targeted immunotherapy, wherein the samples were obtained at separate pre-determined time points; (b) detecting CTCs in the liquid biopsy samples; (c) calculating what proportion of CTCs in the liquid biopsy samples express PD-L1; and (c) comparing the proportion of CTCs that express PD-L1 between the liquid biopsy samples to monitor the expression of PD-L1. Monitoring the expression of PD-L1 on circulating tumor cells (CTCs) of a subject undergoing PD-L1 targeted immunotherapy may inform a decision on continuing the PD-L1 targeted immunotherapy. A decrease of PD-L1 expression on the CTCs over time may be prognostic of a decreased response to PD-L1 targeted immunotherapy. Alternatively, a non-significant change of PD-L1 expression on the CTCs over time may be prognostic of a positive response to PD-L1 targeted immunotherapy. PD-L1 targeted immunotherapy can comprise an anti-PD-1 antibody, an anti-PD-L1 antibody, an anti-PD-L2 antibody, an anti-PD-1 RNAi, an anti-PD-L1 RNAi, an anti-PD-L2 RNAi, an anti-PD-1 antisense RNA, an anti-PD-L1 antisense RNA, an anti-PD-L2 antisense RNA, a dominant negative PD-1 protein, a dominant negative PD-L1 protein, or a dominant negative PD-L2 protein. The PD-L1 targeted immunotherapy can further be part of a regimen comprising a second agent; for example, a compound that reduces the expression or activity of cytotoxic T lymphocyte antigen 4 (CTLA-4) or B and T lymphocyte attenuator (BTLA), for example, an anti-CTLA-4 antibody, an anti-BTLA antibody, an anti-CD28 antibody, an anti-ICOS antibody, an anti-ICOS-L antibody, an anti-B7-1 antibody, an anti-B7-2 antibody, an anti-B7-H3 antibody, or an anti-B7-H4 antibody.

The present invention provides a method for determining if a subject afflicted with bladder cancer is at risk for metastasis, wherein the method comprises:

(a) detecting CTCs that express PD-L1 in a liquid biopsy sample from the subject afflicted with bladder cancer;
(b) calculating what proportion of CTCs in the liquid biopsy that express PD-L1 is cytokeratin (CK)-negative; and
(c) identifying the subject as a candidate at risk for metastasis based on an assessment that the proportion of CK-negative CTCs in the liquid biopsy that express PD-L1 exceeds a predetermined threshold level.

The methods disclosed herein provide predictive indicators for response to PD-L1 targeted immunotherapy. Identification of PD-L1 on CTCs enables early and ongoing opportunities for therapeutic intervention and adjustments throughout a the clinical progression of the cancer.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claim the singular forms "a", "an", and "the" include plural references, unless the content clearly dictates otherwise, and are used interchangeably with "at least one" and "one or more."

As used herein, the terms "comprises", "comprising", "includes", "including ", "contains" , "containing", and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

As used herein, the term "providing" used in the context of a liquid biopsy sample is meant to encompass any and all means of obtaining the sample. The term encompasses all direct and indirect means that result in the presence of the sample in the context of practicing the claimed methods.

PD-L1 as well as other components of the PD-1/PD-L1 signaling pathway can serve as biomarkers in methods for determining if a subject afflicted with cancer is a candidate for PD-L1 targeted immunotherapy. A "biomarker" is any molecule, property, characteristic or aspect that can be measured and correlated with the probability for response to PD-L1 targeted immunotherapy. The term further encompasses any property, characteristic, feature or aspect of a CTC that can be measured and correlated in connection with predicting a response to PD-L1 targeted immunotherapy. For a CTC biomarker, such a measurable feature can include, for example, the presence, absence, or concentration of the biomarker, or a subtype thereof, in the biological sample, an altered immunofluorescent and/or morphological phenotype, such as, for example, nuclear detail, nuclear contour, presence or absence of nucleoli, quality of cytoplasm, quantity of cytoplasm, intensity of immunofluorescent staining patterns in comparison to the appropriate control subjects, and/or the presence or degree of heterogeneity of phenotypes observed for a CTC biomarker. In addition to CTC biomarkers, biomarkers can further include risk indicia including, for example, age, family history, race and diet.

The term "patient" as used herein preferably refers to a human, but also encompasses other mammals. It is noted that, as used herein, the terms "organism", "individual", "subject", or "patient" are used as synonyms and interchangeably.

As used in the compositions and methods described herein, the term "cancer" refers to or describes the physiological condition in mammals that is typically characterized by unregulated cell growth. More specifically, as used herein, the term "cancer" means any cancer in which tumor progression depends on fibroblasts expressing FAP. According to the invention, the cancer is bladder cancer. In one embodiment, the bladder cancer is an epithelial cancer. alternative embodiment, the bladder cancer is an "early stage" cancer. In still another embodiment, the bladder cancer is a "late stage" cancer. The term "tumor" as used herein, refers to all neoplastic cell growth and proliferation, whether malignant or benign, and all pre-cancerous and cancerous cells and tissues. The cancer can be a lymphoproliferative cancer, for example, a precursor B lymphoblastic leukemia/lymphoblastic lymphoma, a B cell non-Hodgkin lymphomas of follicular origin, a Hodgkin lymphoma precursor T cell lymphoblastic leukemia/lymphoblastic lymphoma, a neoplasm of immature T cells, a neoplasm of peripheral, post-thymic T cells, a T cell prolymphocytic leukemia, a peripheral T cell lymphoma, an unspecified, anaplastic large cell lymphoma, an adult T cell leukemia/lymphoma, a chronic lymphocytic leukemia, a mantle cell lymphoma, a follicular lymphoma, a marginal zone lymphoma, a hairy cell leukemia, a diffuse large B cell lymphoma, a Burkitt lymphoma, a lymphoplasmacytic lymphoma, a precursor T lymphoblastic leukemia/lymphoblastic lymphoma, a T cell prolymphocytic leukemia, an angioimmunoblastic lymphoma, or a nodular lymphocyte predominant Hodgkin lymphoma. The method can be performed in relation to any cancer type in which PD-L1 is increased or decreased relative to a threshold level of the biomarker.

As used herein, the term "circulating tumor cell" or "CTC" is meant to encompass any rare cell that is present in a biological sample and that is related to cancer. CTCs, which can be present as single cells or in clusters of CTCs, are often epithelial cells shed from solid tumors found in very low concentrations in the circulation of patients.

As used herein, a "traditional CTC" refers to a single CTC that is cytokeratin positive, CD45 negative, contains a DAPI nucleus, and is morphologically distinct from surrounding white blood cells.

As used herein, a "non-traditional CTC" refers to a CTC that differs from a traditional CTC in at least one characteristic.

In some embodiments, the frequency of PD-L1 expressing CTCs is a biomarker useful for practicing the methods of the invention. As disclosed herein, the presence of PD-L1 on CTCs can predict whether a patient responds to PD-L1 targeted therapy.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

In particular embodiments, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophils, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulations that are distinguishable by methods well known in the art (e.g., FACS, immunohistochemistry). For example, a blood sample can contain populations of non-nucleated cells, such as erythrocytes (e.g., 4-5 million/$\mu$l) or platelets (150,000-400,000 cells/$\mu$l), and populations of nucleated cells such as WBCs (e.g., 4,500 - 10,000 cells/$\mu$l), CECs or CTCs (circulating tumor cells; *e.g.,* 2-800 cells/ $\mu$l). WBCs may contain cellular subpopulations of, e.g., neutrophils (2,500-8,000 cells/$\mu$l), lymphocytes (1,000-4,000 cells/$\mu$l), monocytes (100-700 cells/$\mu$l), eosinophils (50-500 cells/$\mu$l), basophils (25 - 100 cells/$\mu$l) and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

In some embodiments, the sample is a blood sample obtained from a healthy subject or a subject deemed to be at high risk for cancer or metastasis of existing cancer based on art known clinically established criteria including, for example, age, race, and family history. In some embodiments, the blood sample is from a subject who has been diagnosed with cancer based on tissue or liquid biopsy and/or surgery or clinical grounds. In some embodiments, the blood sample is obtained from a subject showing a clinical manifestation of cancer and/or well known in the art or who presents with any of the known risk factors for a particular cancer. According to the invention, the cancer is bladder cancer. In some embodiments, the cancer is urothelial bladder cancer.

As used herein in the context of generating CTC data, the term "direct analysis" means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTC, including subtypes of CTCs described herein, that allows for identification of phenotypes and heterogeneity that cannot be achieved with other CTC detection methods and that enables the analysis of biomarkers in the context of the claimed method.

In some disclosed aspects, the methods for determining if a patient afflicted with bladder cancer is a candidate for PD-L1 targeted immunotherapy can further encompass individual patient risk factors and imaging data, which includes any form of imaging modality known and used in the art, for example and without limitation, X-ray computed tomography (CT), ultrasound, positron emission tomography (PET), electrical impedance tomography and magnetic resonance (MRI). It is understood that one skilled in the art can select an imaging modality based on a variety of art known criteria. As described herein, the methods of the invention can encompass one or more pieces of imaging data. In the methods disclosed herein, and one or more individual risk factors can be selected from the group consisting of age, race, and family history. It is understood that one skilled in the art can select additional individual risk factors based on a variety of art known criteria. As described herein, the methods of the invention can encompass one or more individual risk factors. Accordingly, biomarkers can include imaging data, individual risk factors and CTC data. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins) as well as portions or fragments of a biological molecule.

CTC data can include morphological, genetic, epigenetic features and immunofluorescent features. As will be understood by those skilled in the art, biomarkers can include a biological molecule, or a fragment of a biological molecule, the change and/or the detection of which can be correlated, individually or combined with other measurable features, with cancer. CTCs, which can be present as a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, e.g., an abundance of less than 1:5,000, 1:10,000, 1:30,000, 1:50:000, 1:100,000, 1:300,000, 1:500,000, or 1:1,000,000. In some embodiments, a CTC has an abundance of 1:50:000 to 1:100,000 in the cell population.

The samples of this disclosure may be obtained by any means, including, e.g., by means of solid tissue biopsy or fluid biopsy (*see, e.g.,* Marrinucci D. et 2012, Phys. Biol. 9(1):016003). Briefly, in particular embodiments, the method can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (e.g., procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

In some embodiments, the methods of this disclosure comprise an intitial step of obtaining a white blood cell (WBC) count for the blood sample. In certain embodiments, the WBC count is obtained by using a HemoCue® WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, e.g., in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 $\mu$m. In some embodiments, the material is a film. In some embodiment, the material is a glass slide. In certain embodiments, the method encompasses an initial step of depositing nucleated cells from the blood sample as a monolayer on a glass slide. The glass slide can be coated to allow maximal retention of live cells (*See, e.g.,* Marrinucci D. et 2012, Phys. Biol. 9(1):016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embodiments, the identification of CTCs further comprises comparing the intensity of pan cytokeratin fluorescent staining to surrounding nucleated cells. In some embodiments, the CTC data is generated by fluorescent scanning microscopy to detect immunofluorescent staining of nucleated cells in a blood sample (see, Marrinucci D. et al., 2012, Phys. Biol. 9(1):016003).

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). The CTCs may comprise distinct immunofluorescent staining from surrounding nucleated cells.

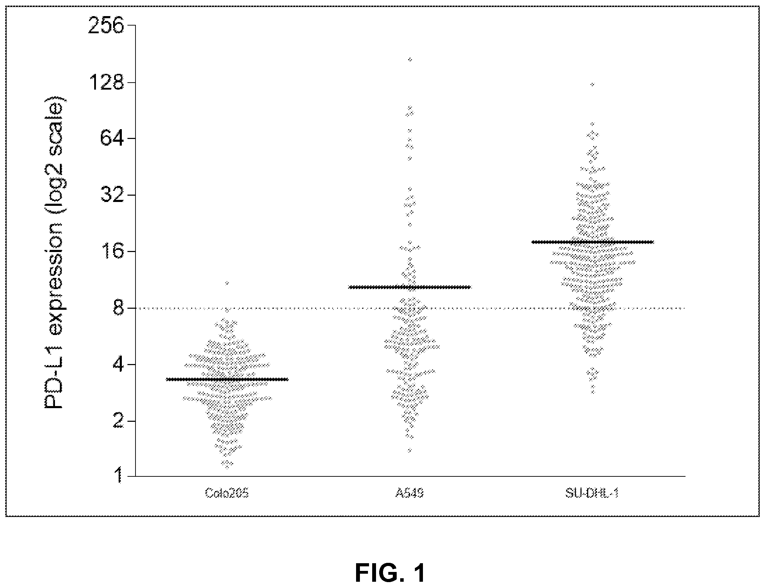

In further disclosed aspects, the CTC data includes traditional CTCs also known as high definition CTCs (HD-CTCs). Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described (Figure 1). Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprising DAPI (+), CK (+) and CD 45 (-) cells, the methods disclosed can be practiced with any other biomarkers that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide.

A person skilled in the art will appreciate that a number of methods can be used to generate CTC data, including microscopy based approaches, including fluorescence scanning microscopy (*see, e.g.,* Marrinucci D. et al., 2012, Phys. Biol. 9(1):016003), sequencing approaches, mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000). A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

Nucleic acid sequencing technologies are suitable methods for analysis of gene expression. The principle underlying these methods is that the number of times a cDNA sequence is detected in a sample is directly related to the relative expression of the RNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

A person of skill in the art will further appreciate that the presence or absence of biomarkers may be detected using any class of marker-specific binding reagents known in the art, including, e.g., antibodies, aptamers, fusion proteins, including protein receptor or protein ligand components, or biomarker-specific small molecule binders. In some embodiments, the presence or absence of CK or CD45 is determined by an antibody. The skilled person will further appreciate that the presence or absence of biomarkers can be measured by evaluating a chromosome copy number change at a chromosome locus of a biomarker. Genomic biomarkers can be identified by any technique such as, for example, comparative genomic hybridization (CGH), or by single nucleotide polymorphism arrays (genotyping microarrays) of cell lines, such as cancer cells. A bioinformatics approach can identify regions of chromosomal aberrations that discriminate between cell line groups and that are indicative of the biomarker, using appropriate copy number thresholds for amplifications and deletions in addition to further analysis using techniques such as qPCR or in situ hybridization. Nucleic acid assay methods for detection of chromosomal DNA copy number changes include: (i) in situ hybridization assays to intact tissue or cellular samples, (ii) microarray hybridization assays to chromosomal DNA extracted from a tissue sample, and (iii) polymerase chain reaction (PCR) or other amplification assays to chromosomal DNA extracted from a tissue sample. Assays using synthetic analogs of nucleic acids, such as peptide nucleic acids, in any of these formats can also be used.

The biomarker may be detected through hybridization assays using detectably labeled nucleic acid-based probes, such as deoxyribonucleic acid (DNA) probes or protein nucleic acid (PNA) probes, or unlabeled primers which are designed/selected to hybridize to the specific designed chromosomal target. The unlabeled primers are used in amplification assays, such as by polymerase chain reaction (PCR), in which after primer binding, a polymerase amplifies the target nucleic acid sequence for subsequent detection. The detection probes used in PCR or other amplification assays are preferably fluorescent, and still more preferably, useful in "real-time PCR". Fluorescent labels are also preferred for use in situ hybridization but other detectable labels commonly used in hybridization techniques, e.g., enzymatic, chromogenic and isotopic labels, can also be used. Useful probe labeling techniques are described in Molecular Cytogenetics: Protocols and Applications, Y.-S. Fan, Ed., Chap. 2, "Labeling Fluorescence In Situ Hybridization Probes for Genomic Targets", L. Morrison et al., p. 21-40, Humana Press, Copyright, 2002 Detecting the genomic biomarkers by microarray analysis, these probe labeling techniques are applied to label a chromosomal DNA extract from a patient sample, which is then hybridized to the microarray.

A biomarker protein may be detected though immunological means or other protein assays. Protein assay methods useful in the invention to measure biomarker levels may comprise (i) immunoassay methods involving binding of a labeled antibody or protein to the expressed biomarker, (ii) mass spectrometry methods to determine expressed biomarker, and (iii) proteomic based or "protein chip" assays for the expressed biomarker. Useful immunoassay methods include both solution phase assays conducted using any format known in the art, such as, but not limited to, an ELISA format, a sandwich format, a competitive inhibition format (including both forward or reverse competitive inhibition assays) or a fluorescence polarization format, and solid phase assays such as immunohistochemistry (referred to as "IHC").

The antibodies used bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used for direct or indirect detection of the biomarkers when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with the selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green™, rhodamine, Texas red, tetramethylrhodamine isothiocynate (TRITC), Cy3, Cy5, Alexa Fluor® 647, Alexa Fluor® 555, Alexa Fluor® 488), fluorescent markers (*e.g.,* green fluorescent protein (GFP), phycoerythrin, etc.), enzymes (e.g., luciferase, horseradish peroxidase, alkaline phosphatase, etc.), nanoparticles, biotin, digoxigenin, metals, and the like.

For mass spectrometry based analysis, differential tagging with isotopic reagents, e.g., isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of $^{125}$I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells. In some embodiments, the immunofluorescent makers comprise a marker specific for white blood cells (WBCs). In some embodiments, one or more of the immunofluorescent markers comprise CD 45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining the presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some embodiments, the method is performed by fluorescent scanning microscopy. In certain embodiments, the microscopic method provides high-resolution images of CTCs and their surrounding WBCs (see, e.g., Marrinucci D. et al., 2012, Phys. Biol. 9(1):016003). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some disclosed aspects, CTC data includes detecting one or more biomarkers, for example, CK and CD 45. A biomarker is considered "present" in a cell if it is detectable above the background noise of the respective detection method used (e.g., 2-fold, 3-fold, 5-fold, or 10-fold higher than the background; e.g., $2\sigma$ or $3\sigma$ over background). In some embodiments, a biomarker is considered "absent" if it is not detectable above the background noise of the detection method used (e.g., <1.5-fold or <2.0-fold higher than the background signal; e.g., $<1.5\sigma$ or $<2.0\sigma$ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (i.e., the marker is considered present) if its fluorescent signal for the respective marker is significantly higher than the fixed background signal (e.g., 2-fold, 3-fold, 5-fold, or 10-fold higher than the background; e.g., $2\sigma$ or $3\sigma$ over background). For example, a nucleated cell is considered CD 45 positive (CD 45$^+$) if its fluorescent signal for CD 45 is significantly higher than the background signal. A cell is considered negative for an immunofluorescent marker (i.e., the marker is considered absent) if the cell's fluorescence signal for the respective marker is not significantly above the background signal (e.g., <1.5-fold or <2.0-fold higher than the background signal; e.g., $<1.5\sigma$ or $<2.0\sigma$ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments, generation of the CTC data comprises enumeration of CTCs that are present in the blood sample. In some embodiments, the methods encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments, generation of the CTC data comprises detecting distinct subtypes of CTCs, including non-traditional CTCs. In some embodiments, the methods encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods encompass detection of at least 1 CTC cluster/mL of blood.

The disclosed methods for determining if a patient afflicted with cancer is a candidate for PD-L1 targeted immunotherapy may encompass the use of a predictive model. In some examples, the disclosed methods for determining if a patient afflicted with cancer is a candidate for PD-L1 targeted immunotherapy encompass comparing a measurable feature with a reference feature. As those skilled in the art can appreciate, such comparison can be a direct comparison to the reference feature or an indirect comparison where the reference feature has been incorporated into the predictive model. In further examples, analyzing a measurable feature to determine if a patient afflicted with cancer is a candidate for PD-L1 targeted immunotherapy encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular examples, the analysis comprises logistic regression. In additional examples, the determination that a patient afflicted with cancer is a candidate for PD-L1 targeted immunotherapy is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g., AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. A desired quality threshold may be a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, e.g. log-transformed, Box-Cox transformed (Box and Cox, Royal Stat. Soc., Series B, 26:211-246(1964)). The data are then put into a predictive model, which will classify the sample according to the state. The resulting information can be communicated to a patient or health care provider.

In some embodiments, the method has a specificity of >60%, >70%, >80%, >90% or higher. In additional embodiment, has a specificity >90% at a classification threshold of 7.5 CTCs/mL of blood.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

Describe herein are kits for the measurement of biomarker levels that comprise containers containing at least one labeled probe, protein, or antibody specific for binding to at least one of the expressed biomarkers in a sample. These kits may also include containers with other associated reagents for the assay. A kit may comprise containers containing a labeled monoclonal antibody or nucleic acid probe for binding to a biomarker and at least one calibrator composition. The kit can further comprise components necessary for detecting the detectable label (e.g., an enzyme or a substrate). The kit can also contain a control sample or a series of control samples which can be assayed and compared to the test sample. Each component of the kit can be enclosed within an individual container and all of the various containers can be within a single package, along with instructions for interpreting the results of the assays performed using the kit.

The following examples are provided by way of illustration, not limitation.

**EXAMPLES**

**Example 1.** Characterization of PD-L1 Expression

Sample evaluation for CTCs was performed as reported previously using the Epic Sciences Platform. Marrinucci et al. Phys Biol 9:016003, 2012. The Epic CTC collection and detection process, which flows as follows: (1) Blood lysed, nucleated cells from blood sample placed onto slides; (2) Slides stored in -80°C biorepository; (3) Slides stained with CK, CD45, DAPI and AR; (4) Slides scanned; (5) Multi-parametric digital pathology algorithms run, and (6) Software and human reader confirmation of CTCs & quantitation of biomarker expression.

Blood samples underwent hemolysis, centrifugation, re-suspension and plating onto slides, followed by -80⁰C storage. Prior to analysis, slides were thawed, labeled by immunofluorescence (pan cytokeratin, CD45, DAPI and PD-L1) and imaged by automated fluoroscopy then manual validation by a pathologist-trained technician (MSL). Marrinucci et al. Phys Biol 9:016003, 2012. DAPI (+), CK (+) and CD45 (-) intensities were categorized as features during CTC enumeration as previously described.

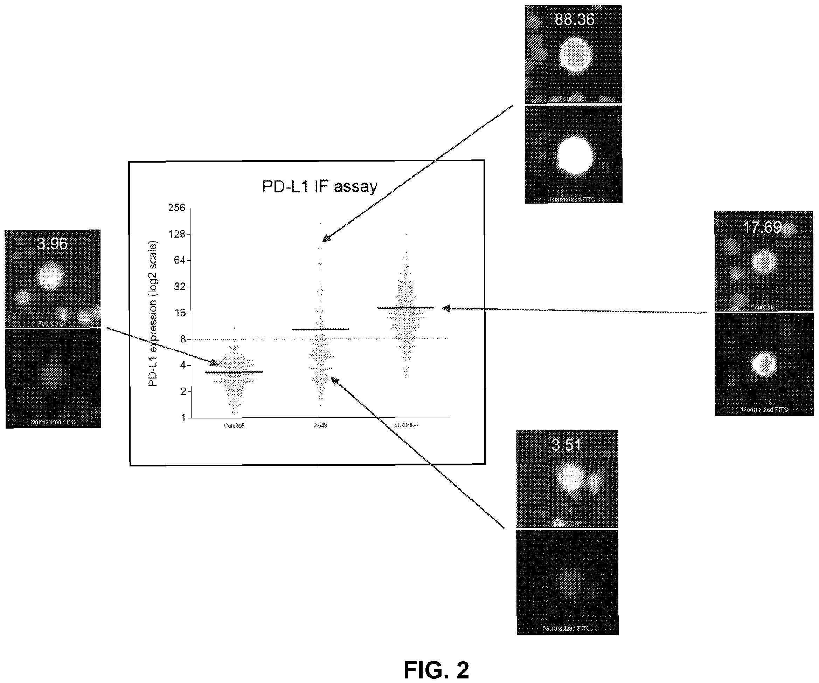

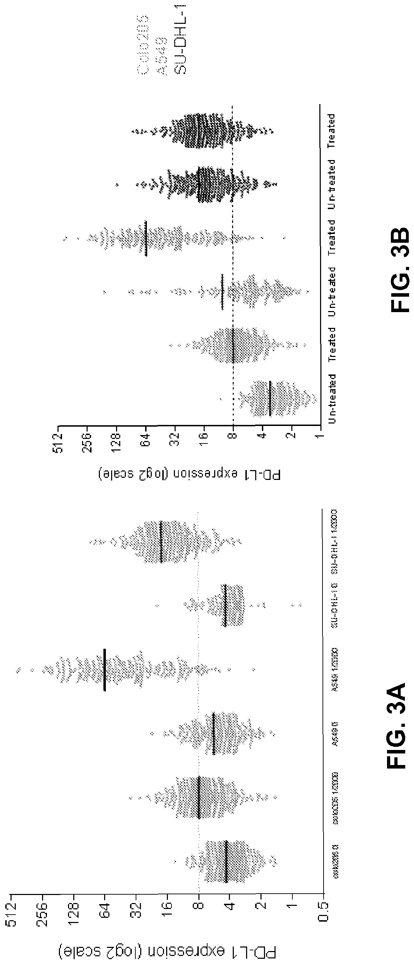

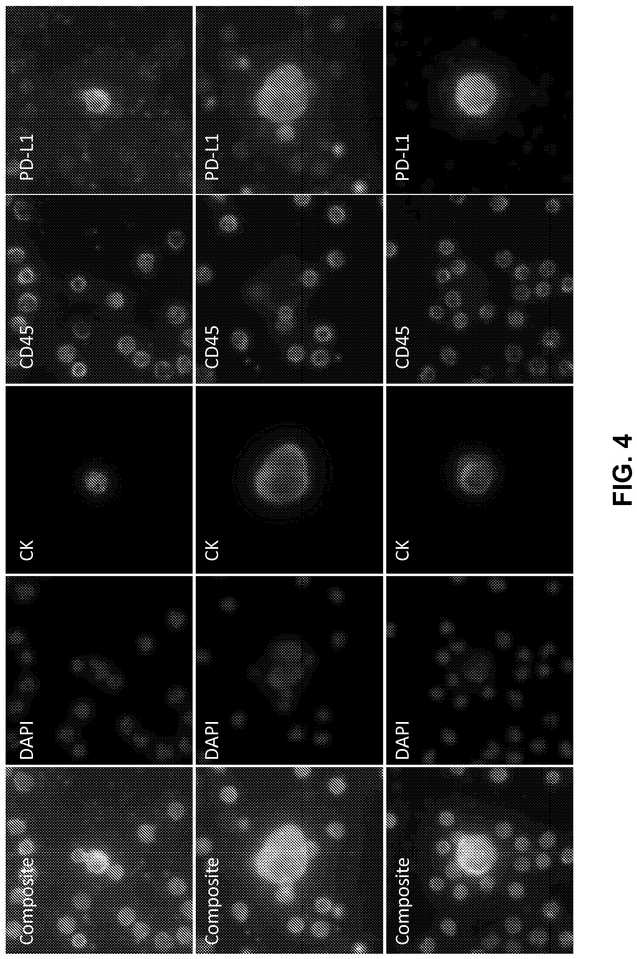

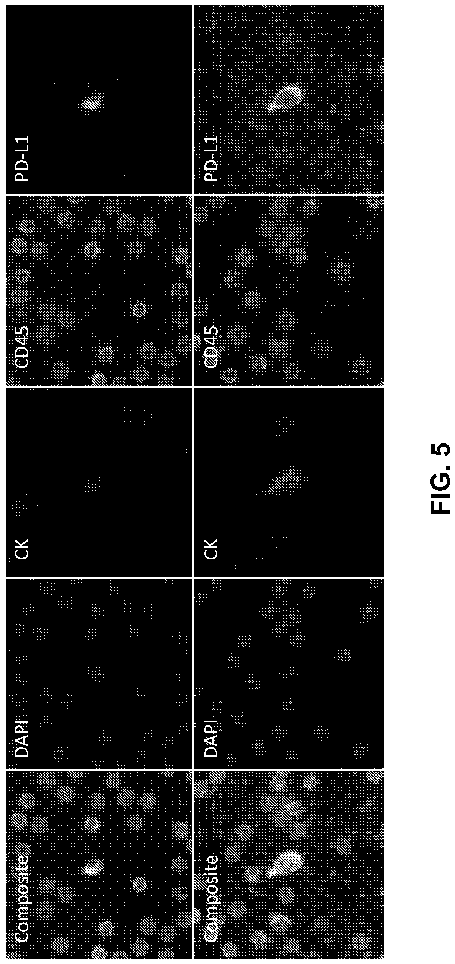

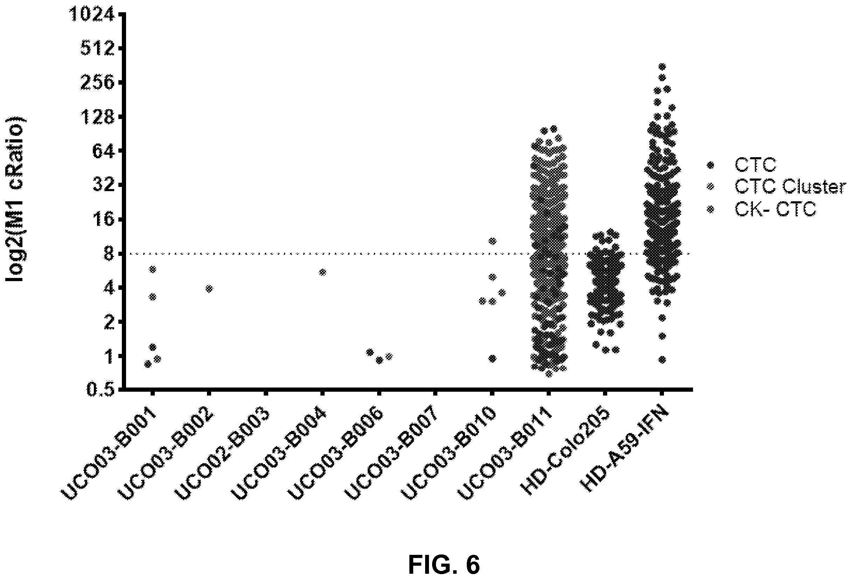

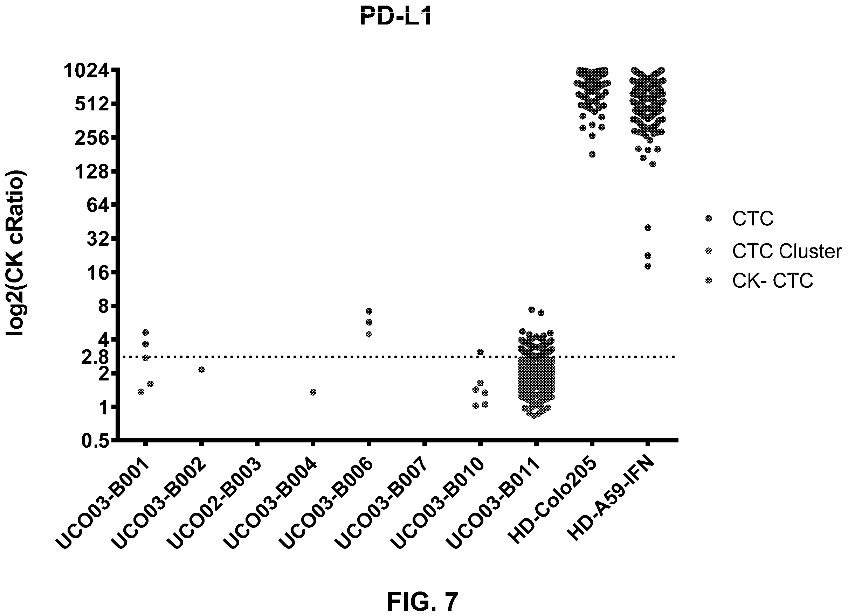

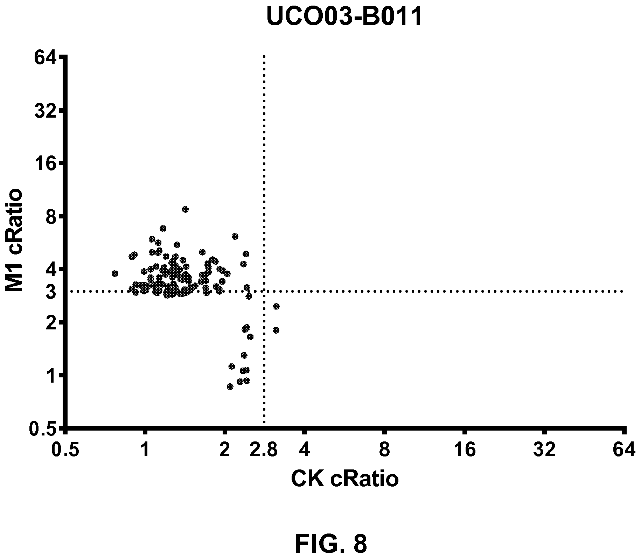

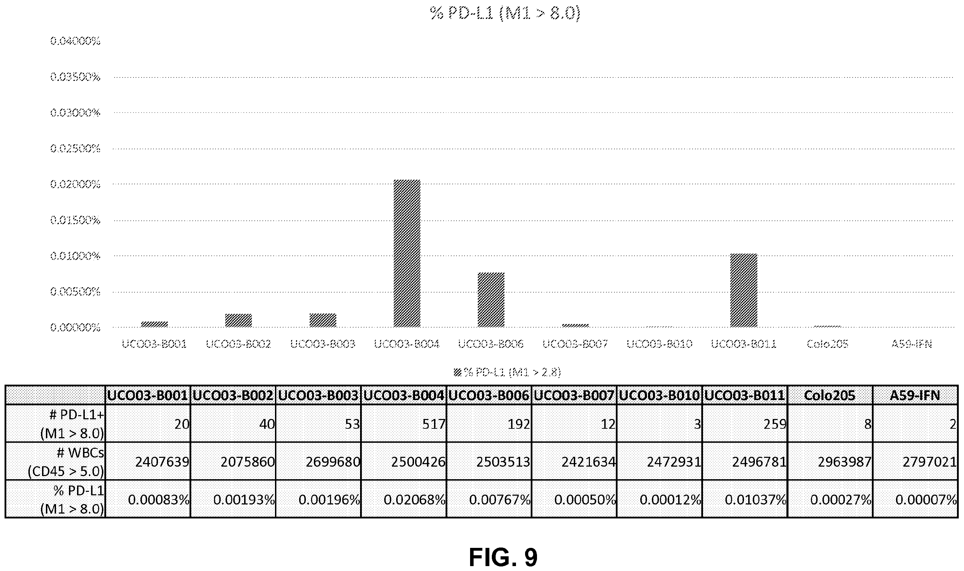

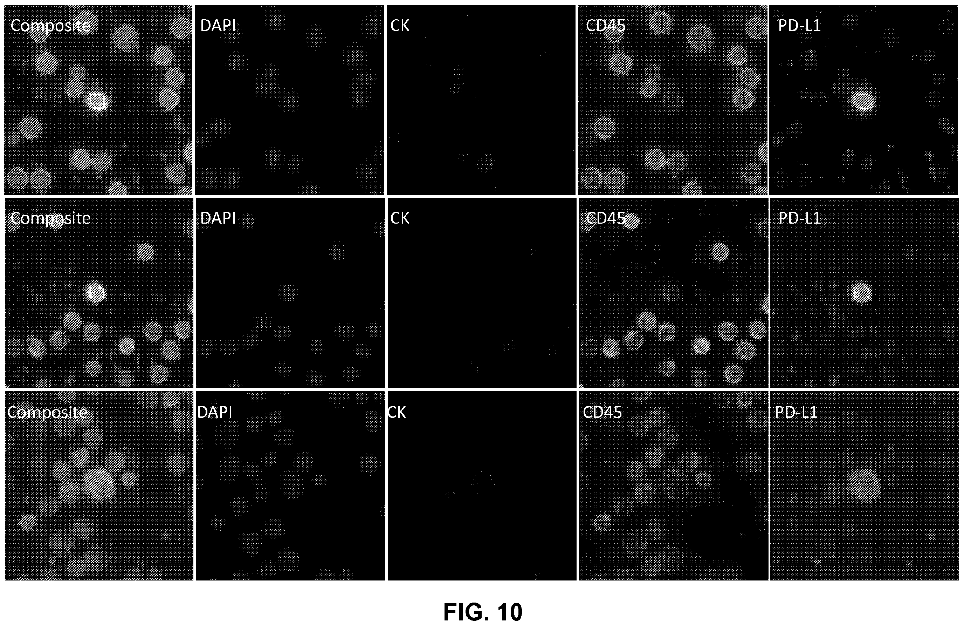

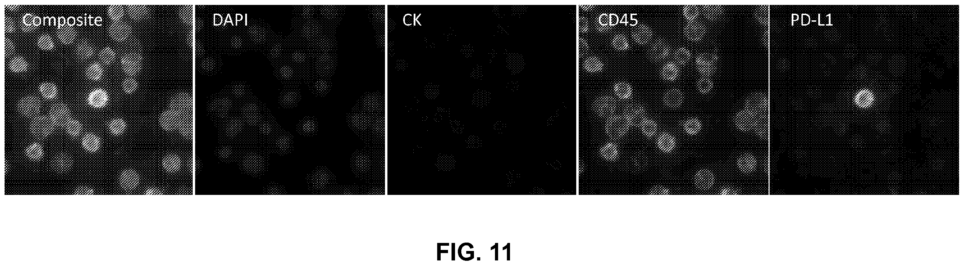

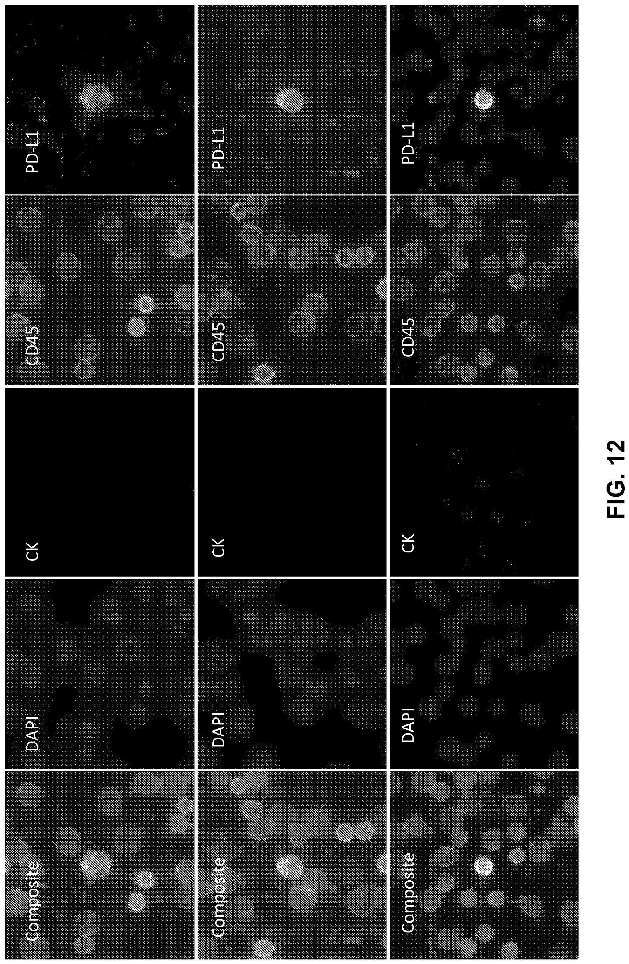

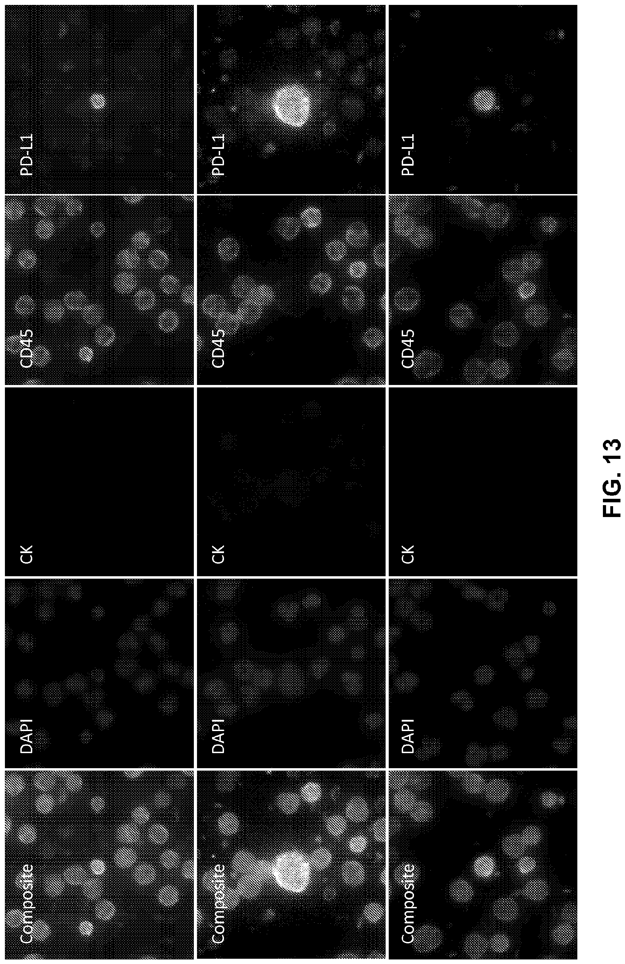

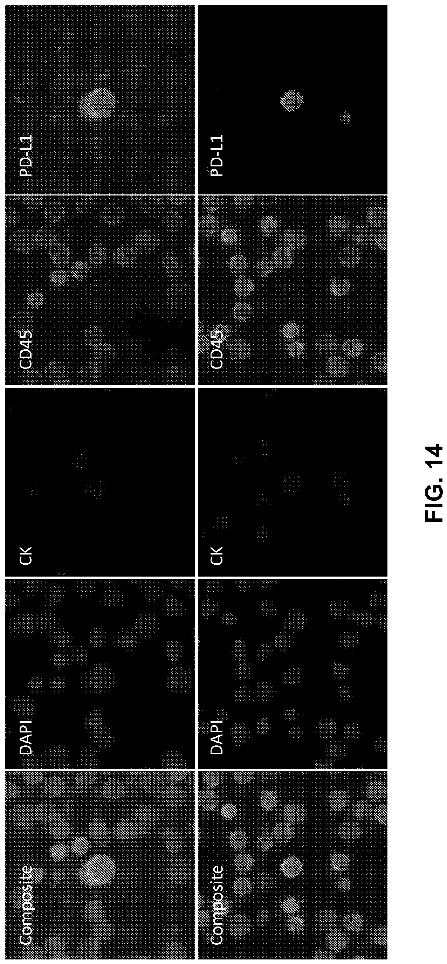

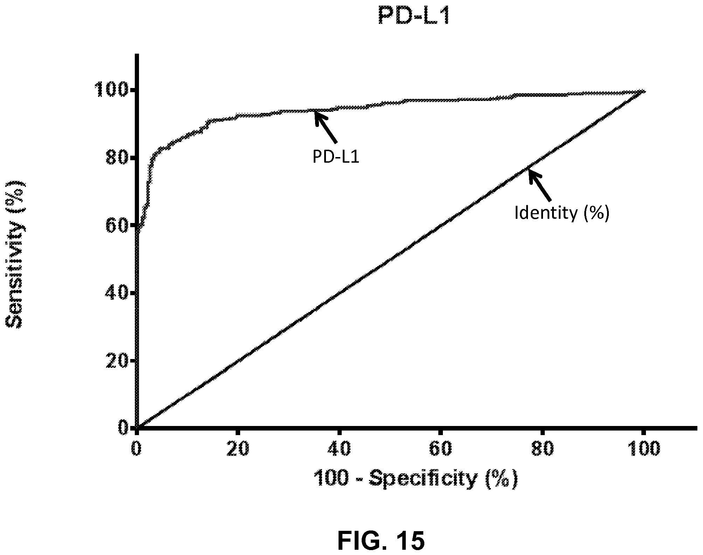

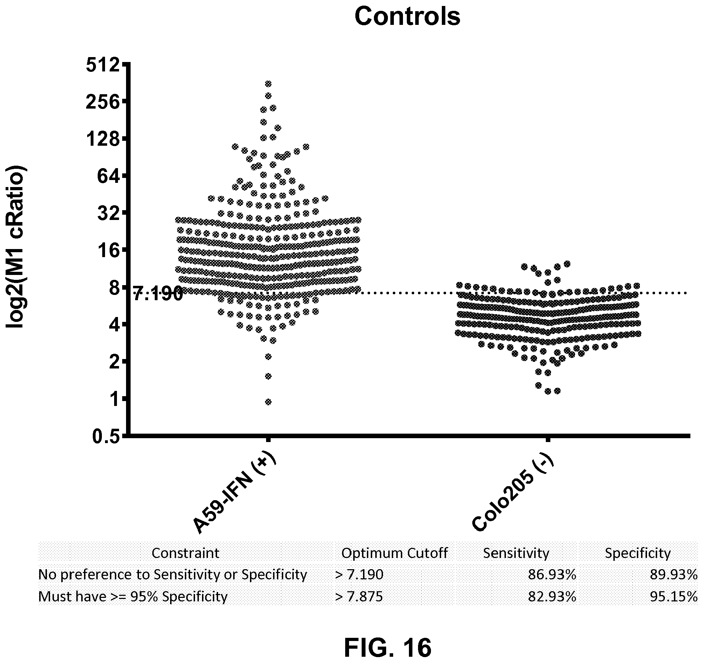

More specifically, peripheral blood sample was collected in Cell-free DNA BCT (Streck, Omaha, NE, USA) and shipped immediately to Epic Sciences (San Diego, CA, USA) at ambient temperature. Upon receipt, red blood cells were lysed and nucleated cells were dispensed onto glass microscope slides as previously described (Marrinucci et al. Hum Pathol 38(3): 514-519 (2007); Marrinucci et al. Arch Pathol Lab Med 133(9):1468-1471 (2009); Mikolajczyk et al. J Oncol 2011: 252361. (2011); Marrinucci et al. Phys Biol 9(1):016003 (2012); Werner et al. J Circ Biomark 4: 3 (2015)) and stored at -80 °C until staining. The millilitre equivalent of blood plated per slide was calculated based upon the sample's white blood cell count and the volume of post-RBC lysis cell suspension used. Circulating tumour cells were identified by immunofluorescence, as described (Marrinucci et al, 2007, *supra;* Marrinucci et al, 2009, *supra;* Mikolajczyk et al, 2011, *supra;* Marrinucci et al, 2012, *supra;* Werner et al, 2015, *supra).* Figures 1 through 16 and the corresponding brief descriptions of the drawings describe further experimental details.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

## Claims

1. A method for determining if a subject afflicted with bladder cancer is at risk for metastasis, wherein the method comprises:

    (a) detecting circulating tumor cells (CTCs) that express PD-L1 in a liquid biopsy sample from the subject afflicted with bladder cancer;
    (b) calculating what proportion of CTCs in the liquid biopsy that express PD-L1 is cytokeratin (CK)-negative; and
    (c) identifying the subject as a candidate at risk for metastasis based on an assessment that the proportion of CK-negative CTCs in the liquid biopsy that express PD-L1 exceeds a predetermined threshold level.

## Patentansprüche

1. Verfahren zum Bestimmen, ob ein an Blasenkrebs erkranktes Subjekt einem Risiko von Metastasenbildung unterliegt, wobei das Verfahren Folgendes umfasst:

    (a) Detektieren zirkulierender Tumorzellen (CTCs), die PD-L1 exprimieren, in einer Flüssigkeitsbiopsieprobe von dem Subjekt, das an Blasenkrebs erkrankt ist;
    (b) Berechnen, welcher Anteil von CTCs in der Flüssigkeitsbiopsie, die PD-L1 exprimieren, Cytokeratin (CK)-negativ ist; und
    (c) Identifizieren des Subjekts als einen Kandidaten, der dem Risiko von Metastasenbildung unterliegt, basierend auf einer Beurteilung, dass der Anteil CK-negativer CTCs in der Flüssigkeitsbiopsie, die PD-L1 exprimieren, einen vorgegebenen Schwellenwert übersteigt.

## Revendications

1. Procédé pour déterminer si un sujet atteint d'un cancer de la vessie présente un risque de métastase, dans lequel le procédé comprend les étapes consistant à :

    (a) détecter des cellules tumorales circulantes (CTC) qui expriment PD-L1 dans un échantillon de biopsie liquide du sujet atteint d'un cancer de la vessie ;
    (b) calculer quelle proportion de CTC dans la biopsie liquide qui expriment PD-L1 est négative à la cytokératine (CK) ; et
    (c) identifier le sujet comme un candidat à risque de métastases sur la base d'une évaluation selon laquelle la proportion de CTC négatives à la CK dans la biopsie liquide qui expriment PD-L1 dépasse un niveau seuil prédéterminé.

**FIG. 1**

FIG. 2

**FIG. 3A**

**FIG. 3B**

**FIG. 4**

**FIG. 5**

**FIG. 6**

EP 3 207 377 B1

**FIG. 7**

FIG. 8

## % PD-L1 (M1 > 8.0)

% PD-L1 (M1 > 2.8)

| | UCO03-B001 | UCO03-B002 | UCO03-B003 | UCO03-B004 | UCO03-B006 | UCO03-B007 | UCO03-B010 | UCO03-B011 | Colo205 | A59-IFN |
|---|---|---|---|---|---|---|---|---|---|---|
| # PD-L1+ (M1 > 8.0) | 20 | 40 | 53 | 517 | 192 | 12 | 3 | 259 | 8 | 2 |
| # WBCs (CD45 > 5.0) | 2407639 | 2075860 | 2699680 | 2500426 | 2503513 | 2421634 | 2472931 | 2496781 | 2963987 | 2797021 |
| % PD-L1 (M1 > 8.0) | 0.00083% | 0.00193% | 0.00196% | 0.02068% | 0.00767% | 0.00050% | 0.00012% | 0.01037% | 0.00027% | 0.00007% |

**FIG. 9**

FIG. 10

FIG. 11

**FIG. 12**

**FIG. 13**

EP 3 207 377 B1

FIG. 14

PD-L1

Identity (%)

PD-L1

Sensitivity (%)

100 - Specificity (%)

FIG. 15

**Controls**

| Constraint | Optimum Cutoff | Sensitivity | Specificity |
|---|---|---|---|
| No preference to Sensitivity or Specificity | > 7.190 | 86.93% | 89.93% |
| Must have >= 95% Specificity | > 7.875 | 82.93% | 95.15% |

**FIG. 16**

EP 3 207 377 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CRISTOFANILLI et al.** *N Engl J Med,* 2004, vol. 351, 781-91
- **MSAOUEL ; KOUTSILIERIS.** *BMC Cancer,* 2011, vol. 11, 336
- **MARRINUCCI D.** *Phys. Biol.,* 2012, vol. 9 (1), 016003
- **MARRINUCCI D. et al.** *Phys. Biol.,* 2012, vol. 9 (1), 016003
- **NIEVA et al.** *Phys Biol,* 2012, vol. 9, 016004
- **PRICE ; NEWMAN.** Principles and Practice of Immunoassay. Grove's Dictionaries, 1997
- **GOSLING.** Immunoassays: A Practical Approach. Oxford University Press, 2000
- **SELF et al.** *Curr. Opin. Biotechnol.,* 1996, vol. 7, 60-65
- **JOHN R. CROWTHER.** The ELISA Guidebook. Humana Press, 2000
- An Introduction to Radioimmunoassay and Related Techniques. Elsevier Science, 1995
- **S. BRENNER et al.** *Nature Biotechnology,* 2000, vol. 18 (6), 630-634
- **J. MARIONI.** *Genome Research,* 2008, vol. 18 (9), 1509-1517
- **R. MORIN.** *Genome Research,* 2008, vol. 18 (4), 610-621
- **A. MORTAZAVI.** *Nature Methods,* 2008, vol. 5 (7), 621-628
- **N. CLOONAN.** *Nature Methods,* 2008, vol. 5 (7), 613-619
- Labeling Fluorescence In Situ Hybridization Probes for Genomic Targets. **L. MORRISON et al.** Molecular Cytogenetics: Protocols and Applications. Humana Press, 2002, 21-40
- **COLIGAN.** *Current Protocols in Immunology,* 1991
- **HARLOW ; LANE.** Antibodies: A Laboratory Manual. 1988
- **GODING.** Monoclonal Antibodies: Principles and Practice. 1986
- **BOX ; COX.** *Royal Stat. Soc., Series B,* 1964, vol. 26, 211-246
- **MARRINUCCI et al.** *Phys Biol,* 2012, vol. 9, 016003
- **MARRINUCCI et al.** *Hum Pathol,* 2007, vol. 38 (3), 514-519
- **MARRINUCCI et al.** *Arch Pathol Lab Med,* 2009, vol. 133 (9), 1468-1471
- **MIKOLAJCZYK et al.** *J Oncol,* 2011, vol. 2011, 252361
- **MARRINUCCI et al.** *Phys Biol,* 2012, vol. 9 (1), 016003
- **WERNER et al.** *J Circ Biomark,* 2015, vol. 4, 3